(19) Europäisches Patentamt
European Patent Office
Office européen des brevets

(11) Publication number: **0 086 563**
**A1**

# (12) EUROPEAN PATENT APPLICATION

(21) Application number: 83300234.8

(22) Date of filing: 18.01.83

(51) Int. Cl.³: **C 07 D 205/08**, C 07 D 403/12, C 07 D 405/12, C 07 D 405/14, C 07 D 409/12, C 07 D 409/14, A 61 K 31/395

(30) Priority: 22.01.82 GB 8201777

(43) Date of publication of application: 24.08.83 Bulletin 83/34

(84) Designated Contracting States: CH DE FR GB IT LI NL

(71) Applicant: BEECHAM GROUP PLC, Beecham House Great West Road, Brentford Middlesex (GB)

(72) Inventor: Branch, Clive Leslie, Thornbury 18 Overdale, Dorking Surrey (GB)
Inventor: Pearson, Michel John, 33 Greenfields Road, Horsham Sussex (GB)

(74) Representative: Hesketh, Alan, Dr. et al, European Patent Attorney Beecham Pharmaceuticals Great Burgh Yew Tree Bottom Road, Epsom, Surrey, KT18 5XQ (GB)

## (54) Antibacterial agents, their preparation and use.

(57) The present invention provides a β-lactam antibiotic having a sulphonic acid substituent or salt thereof at the 1-position and a hydroxymethylsubstituent at the 3-positions.

EP 0 086 563 A1

# ANTIBACTERIAL AGENTS, THEIR PREPARATION AND USE

This invention relates to β-lactam containing antibacterial agents and in particular to a class of monocyclic β-lactams having a 3R-hydroxymethyl substituent. This invention further relates to processes for preparing such compounds and to compositions containing them. These compounds are of use in the treatment of bacterial infection in animals, for example mammals such as man.

The present invention provides a β-lactam antibiotic having a sulphonic acid substituent or salt thereof at the 1-position and a hydroxymethylsubstituent at the 3-position.

Suitably the present invention provides the compounds of the formula (I):

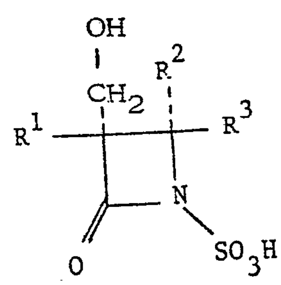

(I)

and salts thereof wherein $R^1$ is an amino, protected amino or carboxylic acylamino group, and $R^2$ and $R^3$ are independently selected from hydrogen or a hydrocarbon group of 1 to 18 carbon atoms.

Suitably $R^1$ is a carboxylic acylamino group such as found in antibacterially active penicillins or cephalosporins. Thus suitable groups $R^1$ include those of the sub-formulae (a), (b), (c), (d) and (e):

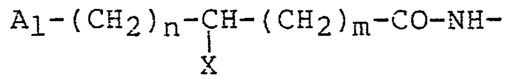 (a)

$A_2-CO-NH-$ (b)

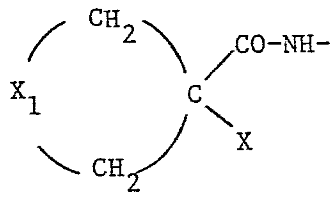 (c)

$A_2-X_2-(CH_2)_n-CO-NH-$ (d)

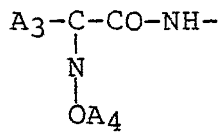 (e)

wherein n is zero, one or two, m is zero, one or two; $A_1$ is $C_{1-6}$ alkyl, $C_{3-6}$ cycloalkyl, $C_{4-7}$ cycloalkenyl, cyclohexadienyl, aryl or heteroaryl such as phenyl, substituted phenyl such as hydroxyphenyl, thienyl or pyridyl; X is a hydrogen, bromine or chlorine atom, or a carboxylic acid, carboxylate ester, sulphonic acid, tetrazolyl, azido, hydroxy, acyloxy, amino, acylamino, heterocyclylamino, ureido, guanidino or acylureido; $A_2$ is an aromatic group such as phenyl, 2,6-dimethoxyphenyl, 2-alkoxy-l-naphthyl, 3-arylisoxazolyl, isothiazolyl or 3-aryl-5-methyl-isoxazolyl group; $X_1$ is a $CH_2OCH_2$, $CH_2SCH_2$ or $(CH_2)_n$ group, $X_2$ is an oxygen or sulphur atom; $A_3$ is an aryl or heteroaryl group such as phenyl, substituted phenyl or aminothiazolyl; and $A_4$ is hydrogen, $C_{1-6}$ alkyl, $C_{3-6}$ cycloalkyl, arylaminocarbonyl, $C_{1-6}$ alkylamino-carbonyl, $C_{1-6}$ alkanoyl, $C_{1-6}$ alkoxycarbonyl, $C_{2-6}$ alkenyl, carboxy$C_{1-6}$alkyl, $C_{1-6}$alkylsulphonyl and di-$C_{1-6}$alkylphosphatomethyl.

More suitably $R^1$ includes groups of the sub-formulae (f) and (g):

$$R^4-\underset{\displaystyle R^5}{\underset{|}{C}}H-CO-NH \qquad (f)$$

$$R^6-\underset{\displaystyle R^7}{\underset{|}{C}}H-CO-NH \qquad (g)$$

wherein $R^4$ is a phenyl, thienyl or phenoxy group; $R^5$ is a hydrogen atom or methyl group; $R^6$ is a phenyl, p-hydroxyphenyl, cyclohexadienyl, or a 5- or 6-membered heteroaryl or heterocyclyl group containing one to three heteroatoms selected from sulphur, oxygen or nitrogen, said group being optionally substituted by one, two or three substituents selected from hydroxy, amino, halo and $C_{1-6}$ alkoxy; and $R^7$ is a hydroxy, amino or carboxylic acid group or a phenyl, methylphenyl, indanyl or $C_{1-6}$ alkyl ester thereof, or is amino, ureido, acylamino or acylureido.

Examples of suitable groups $R^6$ include thienyl, pyridyl, phenyl, p-hydroxyphenyl and aminothiazolyl.

A particularly preferred group of the sub-formula (f) is the phenoxyacetamido group. Another particularly preferred group of the sub-formula (f) is the phenylacetamido group.

Suitably the ureido group in either the sub-formula (c) or sub-formula (g) is of the formula $-NH-CO-NR^8R^9$ wherein $R^8$ is a hydrogen atom or a $C_{1-6}$ alkyl group and $R^9$ is an organic group, or $R^8$ and $R^9$ together with the nitrogen atom to which they are joined form an optionally substituted heteroaryl or

heterocyclyl ring containing 1 or 2 nitrogen atoms.

Suitably $R^9$ is $C_{1-6}$ alkyl or an optionally substituted 5- or 6- membered heteroaryl or heterocyclyl group containing one or two nitrogen atoms.

Suitable substituents for $R^9$ and for the rings formed by $R^8$ and $R^9$ together include one, two or three groups selected from $C_{1-6}$ alkyl, $C_{2-6}$ alkenyl, $C_{2-6}$ alkynyl, $C_{3-6}$ cycloalkyl, optionally substituted phenyl, oxo, hydroxy optionally substituted such as $C_{1-6}$ alkoxy, $C_{2-6}$ alkenyloxy, $C_{3-6}$ cycloalkyloxy or phenoxy, mercapto optionally substituted such as phenylthio or $C_{1-6}$ alkylthio; or amino or substituted amino such as $C_{1-6}$ alkylamino, optionally substituted phenylamino, benzylamino or sulphonylamino for example $C_{1-6}$ alkyl sulphonylamino or p-aminosulphonylphenylamino. Alternatively two substituents on the ring $R^9$ or on the ring formed by $R^8$ and $R^9$ together may form the residue of a further carbocyclic or heterocyclic ring.

Suitably also X may be an acylamino or acylureido group, for example, of the sub-formula (h):

$$-NH-CO-(NH-CO)_p-R^{10} \qquad (h)$$

wherein p is zero or one and $R^{10}$ is a hydrogen atom or an organic group such as aryl, heteroaryl, heterocyclyl, $C_{2-6}$ alkenyl or $C_{1-6}$ alkyl.

Compounds of the formula (I) wherein $R^1$ is amino or protected amino have antibacterial activity but are envisaged mainly as useful intermediates in the

preparation of compounds of the formula (I) wherein $R^1$ is carboxylic acylamino. "Protected amino" means that the amino moiety ($NH_2$-) is protected in conventional manner by a group or groups that may be removed under conventional conditions and would be suitable for use in the processes of this invention. Examples of suitable protected amino groups include hydrocarbyloxy-carbonylamino such as t-butoxycarbonylamino and benzyloxycarbonylamino; and benzylideneamino such as Ph-CH=N-.

Suitably in the compounds of the formula (I) $R^2$ is a hydrogen atom. Suitably also $R^3$ is a hydrogen atom. Thus in a preferred aspect $R^2$ and $R^3$ are each hydrogen.

Alternatively $R^2$ and $R^3$ are independently selected from $C_{1-18}$ hydrocarbons, for example $C_{1-6}$ alkyl, $C_{2-6}$ alkenyl, $C_{2-6}$ alkynyl, $C_{3-8}$ cycloalkyl, aryl, aryl($C_{1-6}$)alkyl, aryl($C_{2-6}$)alkenyl, heteroaryl, heteroaryl($C_{1-6}$)alkyl, heterocyclyl and heterocyclyl ($C_{1-6}$)alkyl, any of such groups being optionally substituted. Suitably the above named heteroaryl and heterocyclyl rings contain not more than 10 ring atoms, up to 4 of which are selected from oxygen, sulphur and nitrogen.

One particularly preferred sub-group of compounds is that of the formula (II):

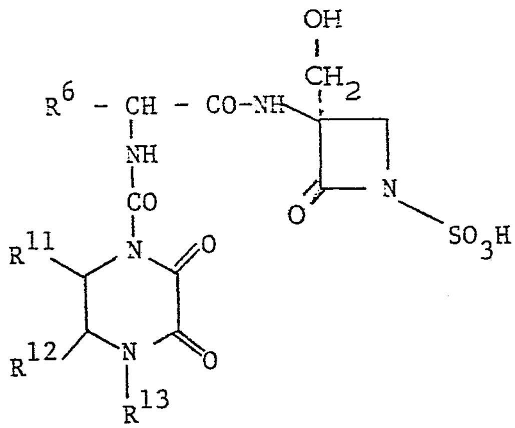

(II)

and salts thereof wherein $R^6$ is as hereinbefore defined, $R^{11}$ and $R^{12}$ are independently selected from hydrogen, $C_{1-6}$ alkyl, halo, amino, hydroxy or $C_{1-6}$ alkoxy, and $R^{13}$ is hydrogen, $C_{1-6}$ alkyl, or aralkyl such as benzyl. Suitably $C_{1-6}$ alkyl groups for the groups $R^{11}$, $R^{12}$ and $R^{13}$ include methyl, ethyl, n- and iso-propyl, and n, sec-, iso- and tert- butyl. Preferably $R^{13}$ is ethyl. Preferably $R^{11}$ and $R^{12}$ are each hydrogen.

A further preferred sub-group of compounds is that of the formula (III):

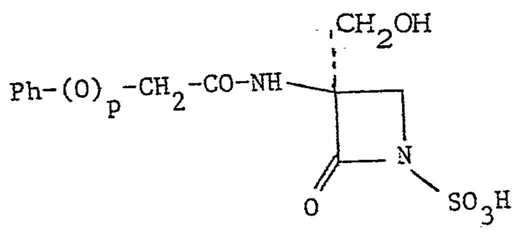

(III)

and salts thereof wherein p is zero or one.

Another preferred sub-group of compounds is that of the formula (IV):

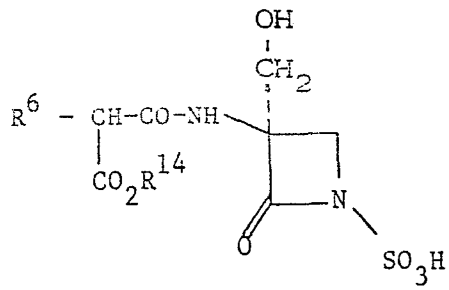

(IV)

and salts thereof wherein $R^{14}$ is a cation, hydrogen atom or a benzyl, phenyl, methylphenyl or indanyl group, and $R^6$ is as hereinbefore defined.

Yet another preferred sub-group of compounds is that of the formula (V):

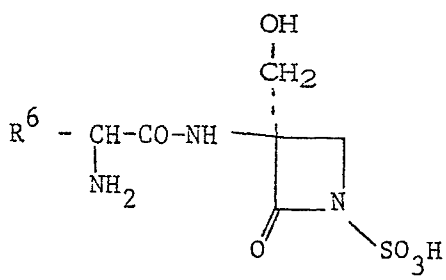

(V)

and salts thereof wherein $R^6$ is as defined hereinabove.

Another class of preferred compounds is that of the formula (VI):

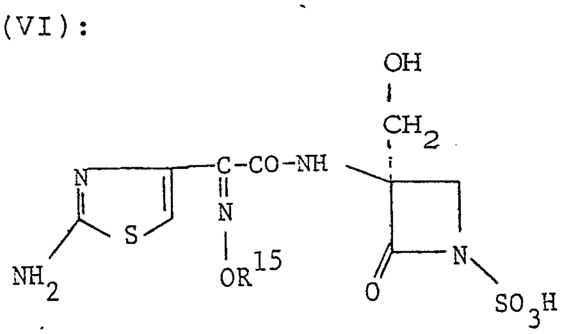

(VI)

and salts thereof wherein $R^{15}$ is hydrogen, $C_{1-6}$ alkyl such as methyl and ethyl, $C_{1-6}$ alkanoyl such as formyl and acetyl, methoxycarbonyl, ethenyl, methanesulphonyl,

carboxy($C_{1-6}$)alkyl such as carboxymethyl, carboxyethyl and 2-carboxyprop-2-yl, or dimethylphosphatomethyl.

The major utility of the compounds of the formulae (I) - (VI) and salts thereof is as pharmaceuticals and accordingly the salts are preferably pharmaceutically acceptable. The compounds of this invention both pharmaceutically acceptable and non-pharmaceutically acceptable may be used as intermediates, for example for the preparation of pharmaceutically acceptable salts of this invention, or in non-pharmaceutical usage such as a disinfectant or paint additive.

Suitable pharmaceutically acceptable salts include metal salts such as alkali metal salts for example sodium and potassium, alkaline earth metal salts such as calcium and magnesium, and ammonium and substituted ammonium salts, for example tetrabutylammonium and pyridinium.

In another aspect of this invention there is provided a pharmaceutical composition which comprises a compound of the formula (I) or a pharmaceutically acceptable salt thereof and a pharmaceutically acceptable carrier.

The composition may be formulated for administration by any route, such as oral topical or parenteral. The compositions may be in the form of tablets, capsules, powders, granules, lozenges, creams or liquid preparations, such as oral or sterile parenteral solutions or suspensions.

Tablets and capsules for administration may be in unit dose presentation form, and may contain

conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be

either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-5000 mg of the active ingredient. The dosage as employed for adult human treatment will preferably range from 100 to 3000 mg per day, for instance 1500 mg per day depending on the route and frequency of administration.

The compound of formula (I) in pharmaceutically acceptable form may be the sole therapeutic agent in the compositions of the invention or a combination with

other antibiotics or with a β-lactamase inhibitor may be employed.

Suitable β-lactamase inhibitors include the compounds of the formula (VII) and pharmaceutically acceptable salts and esters thereof:

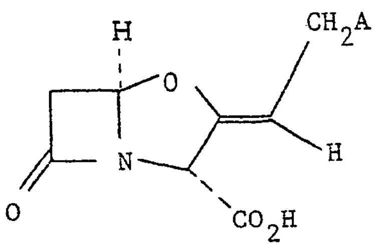

(VII)

wherein A is hydroxy, substituted hydroxy, mercapto, substituted mercapto, amino, mono- or di-hydrocarbyl-substituted amino, or mono- or di-acylamino.

Further suitable β-lactamase inhibitors include penicillanic acid 1,1-dioxide and salts and <u>in-vivo</u> hydrolysable esters thereof, 6β-bromopenicillanic acid and salts and <u>in-vivo</u> hydrolysable esters and 6β-iodopenicillanic acid and salts and <u>in-vivo</u> hydrolysable esters thereof.

Such compositions of this invention comprising a β-lactamase inhibitor are formulated in conventional manner.

In another aspect the present invention provides a process for the preparation of a compound of the present invention or salt thereof which process comprises the sulphonation of a β-lactam having a hydrogen substituent at the 1-position and a hydroxymethyl substituent at the 3 position.

The present invention further provides a process for the preparation of a compound of the formula (I) or a salt thereof which process comprises sulphonation of

a compound of the formula (VIII):

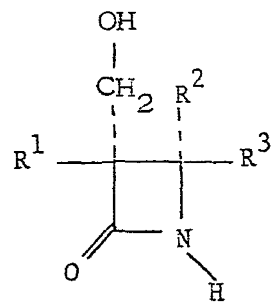

(VIII)

or reactive derivative thereof, wherein $R^1$, $R^2$ and $R^3$ are as hereinbefore defined, and thereafter if necessary:

i) converting a compound of the formula (I) wherein $R^1$ is amino or protected amino to a compound of the formula (I) wherein $R^1$ is carboxylic acylamino,

ii) forming a pharmaceutically acceptable salt.

The sulphonation reaction may be conveniently performed using a sulphur trioxide complex, examples of which include pyridine-sulphur trioxide, dimethylformamide-sulphur trioxide, trimethylamine-sulphur trioxide, lutidine-sulphur trioxide, dioxan-sulphur trioxide and chlorosulphonic acid-sulphur trioxide. A preferred reagent is pyridine-sulphur trioxide.

The sulphonation reaction is performed at a non-extreme temperature such as 0° - 80°C, more suitably 10° - 50°C and conveniently at ambient temperature. Normally the reaction is performed in a solvent, for example an organic solvent such as dimethylformamide, dichloromethane, chloroform, dioxan, tetrahydrofuran, or mixtures thereof. Of these the preferred solvent is dimethylformamide.

It will be appreciated that the sulphonation reaction may lead to the preparation of a specific salt of the β-lactam, for example where the reagent used is pyridine-sulphur trioxide, the salt formed is the pyridinium salt. This may be converted to any other desired salt using conventional methods of ion-replacement, for example using ion-exchange resin, ion-pair extraction, crystallisation and precipitation.

Compounds of the formula (I) wherein $R^1$ is protected amino may be converted to compounds of the formula (I) wherein $R^1$ is carboxylic acylamino via the intermediacy of the compounds of the formula (I) wherein $R^1$ is amino.

Suitable amino-protecting groups include those known in the β-lactam art to be convertible to amino, for example hydrogenolysable groups, such as benzyloxycarbonylamino.

Compounds of the formula (I) wherein $R^1$ is carboxylic acylamino may be prepared by the reaction of a compound of the formula (I) wherein $R^1$ is amino or a derivative thereof that permits N-acylation to take place, with an N-acylating derivative of a carboxylic acid of the formula (IX):

$$R^{16}CO_2H \qquad (IX)$$

wherein $R^{16}$ is an organic group and any reactive group is optionally protected, and thereafter if necessary, removing any protecting group.

Suitable groups which permit acylation to take place and which are optionally present on the amino

group $R^1$ include N-silyl, N-stannyl and N-phosphorus groups, for example trialkylsilyl groups such as trimethylsilyl, trialkyltin groups such as tri-n-butyltin, groups of the formula $-PR^aR^b$ wherein $R^a$ is an alkyl, alkoxy, haloalkyl, aryl, aralkyl, haloalkoxy, aryloxy, aralkyloxy or dialkylamino group, $R^b$ is the same as $R^a$ or is halogen or $R^a$ and $R^b$ together form a ring: suitable such phosphorus groups being $-P(OC_2H_5)_2$, $-P(C_2H_5)_2$, $-\underline{P-OCH_2CH_2O}$.

A reactive N-acylating derivative of the acid (IX) is employed in the above process. Suitable N-acylating derivatives include an acid halide, preferably the acid chloride or bromide. Acylation with an acid halide may be effected in the presence of an acid binding agent for example molecular sieve or a pyridine or a tertiary amine (such as triethylamine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2- alkylene oxide - such as ethylene oxide or propylene oxide. The acylation reaction using an acid halide may be carried out at a temperature in the range -50°C to +50°C, preferably -20°C to +20°C, in aqueous or non-aqueous media such as aqueous acetone, ethyl acetate, dimethylacetamide, dimethylformamide, acetonitrile, dichloromethane, 1,2-dichloroethane, or mixtures thereof.

Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (IX) or a salt thereof with a halogenating (eg. chlorinating or brominating) agent such as phosphorus pentachloride, thionyl chloride or oxalyl chloride.

Alternatively, the N-acylating derivative of the acid (IX) may be a symmetrical or mixed anhydride. Suitable mixed anhydrides are alkoxyformic anhydrides, or anhydrides with, for example carbonic acid mono-esters, trimethyl acetic acid, thioacetic acid, diphenylacetic acid, benzoic acid, phosphorus acids such as phosphoric or phosphorous acids, sulphuric acid or aliphatic or aromatic sulphonic acids such as p-toluenesulphonic acid. The mixed or symmetrical anhydrides may be generated using N-ethoxycarbonyl-2-ethoxyl-2-ethoxy-1,2-dihydroquinoline. When a symmetrical anhydride is employed, the reaction may be carried out in the presence of 2,6-lutidine as catalyst.

Alternatively N-acylating derivatives of acid (IX) are the acid azide, or activated esters such as esters with 2-mercaptopyridine, 2-mercaptothiazoline, cyanomethanol, p-nitrophenol, 2,4-dinitrophenol, thioalcohols such as thiophenol, methanethiol, ethanethiol and propanethiol, halophenols, including pentachlorophenol, monomethoxy- phenol or 8-hydroxyquinoline, N-hydroxysuccinimide or 1-hydroxybenztriazole; or amides such as N-acylsaccharins or N-acylphthalimides; or an alkylidine iminoester prepared by reaction of the acid (IX) with an oxime.

Other reactive N-acylating derivatives of the acid (IX) include the reactive intermediate formed by

reaction *in situ* with a condensing agent such as a carbodiimide, for example N,N-diethyl-, dipropyl- or diisopropylcarbodiimide, N,N'-di-cyclohexylcarbodiimide, or N-ethyl-N'-$\gamma$-dimethylaminopropylcarbodiimide; a suitable carbonyl compound, for example N,N'-carbonyldiimidazole or N,N'-carbonylditriazole; an isoxazolinium salt, for example N-ethyl-5-phenylisoxazolinium-3-sulphonate or N-t-butyl-5-methylisoxazolinium perchlorate; or an N-alkoxycarbonyl-2-alkoxy-1,2-dihydroquinoline, such as N-ethoxycarbonylethoxy-1,2-dihydroquinoline. Other condensing agents include Lewis acids (for example $BBr_3 - C_6H_6$); or a phosphoric acid condensing agent such as diethylphosphorylcyanide. The condensation reaction is preferably carried out in an organic reaction medium, for example methylene chloride, dimethylformamide, acetonitrile, alcohol, benzene, dioxan or tetrahydrofuran.

Preferably in the sulphonation of a compound of the formula (VIII) to provide a compound of the formula (I), and during any subsequent interconversion of a group $R^1$ to another group $R^1$, the 3R-$CH_2OH$ group may be protected, for example, as a silyl derivative removable by hydrolysis, or by fluoride ion or an acylated group removable by hydrogenolysis. Examples of such suitable protecting groups include tertiarybutyldimethylsilyl or benzyloxycarbonyl or a substituted benzyloxycarbonyl such as p-nitrobenzyloxycarbonyl, so that a silyloxy or benzyloxycarbonyloxy group is formed.

In a further aspect of the present invention provides a process for the preparation of a compound of the formula (I) or (VIII) which process comprises the reaction of a compound of the formula (X):

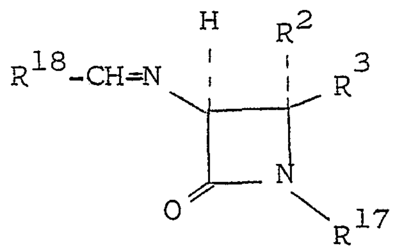

(X)

wherein $R^2$ and $R^3$ are as hereinbefore defined, $R^{17}$ is hydrogen, $SO_3H$ or a protecting group, and $R^{18}$ is an optionally substituted aromatic ring; with formaldehyde; and thereafter, if necessary:

i) optionally protecting the $-CH_2OH$ group;

ii) converting $R^{18}$-CH=N to an amino group;

iii) converting said amino group to a carboxylic acylamino group;

iv) converting a compound wherein $R^{17}$ is not $SO_3H$ to a compound wherein $R^{17}$ is $SO_3H$,

v) removing any protecting groups.

Suitably $R^{18}$ is p-nitrophenyl.

The reaction of the compound of the formula (X) and formaldehyde is performed in the presence of an inorganic or organic base, preferably a tertiary amine or a pyridine type base. Examples of suitable bases include di-isopropylethylamine, di-isopropylcyclohexylamine, sodium hydroxide, potassium carbonate and

sodium hydride. The reaction is suitably performed in an inert aprotic solvent such as dimethylformamide, benzene, toluene and acetonitrile.

Examples of suitable protecting groups for the group $R^{17}$ include those known in the art as being cleavable to provide the -NH-. Mention may be made of silyl groups such as trimethylsilyl, tertiary butyldimethylsilyl and tri-isopropylsilyl. A preferred protecting group is (p-methoxymethoxy)phenyl which is removable by cerium ammonium nitrate. Other protecting groups of interest include those cleavable by methanolysis such as $-C(CO_2R)=O$ (This moiety may be derived from groups of the type $-C(CO_2R)=C(CH_3)_2$). Further suitable protecting groups include $C_{1-6}$ alkoxy and benzyloxy. A further suitable protecting group is 2,4-dimethoxybenzyl which is removable with potassium persulphate.

Compounds of the formula (X) may be prepared by reaction of a compound of the formula (XII):

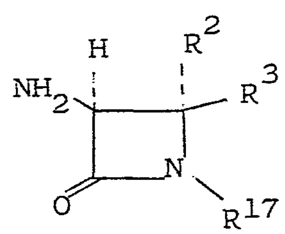

(XII)

wherein $R^2$, $R^3$ and $R^{17}$ are as hereinbefore defined, with a compound of the formula (XIII):

$R^{18}$ - CHO (XIII)

wherein $R^{18}$ is as hereinbefore defined.

Such reaction is conveniently performed in an inert solvent such as benzene, toluene, dimethylformamide or acetonitrile, and may be conducted at ambient or an elevated temperature, for example 15°C to 110°C. This reaction is an equilibrium reaction involving the formation of water, so the reaction is aided by removing the water during the course of the reaction, for example by azeotropic distillation or by the presence of a drying agent.

The compounds of the formula (XII) may be prepared for example by the methods disclosed and discussed in U.K. Patent Application Publication No. 2071650A, or the methods exemplified hereinafter which methods are summarised by the following Schemes.

SCHEME 1

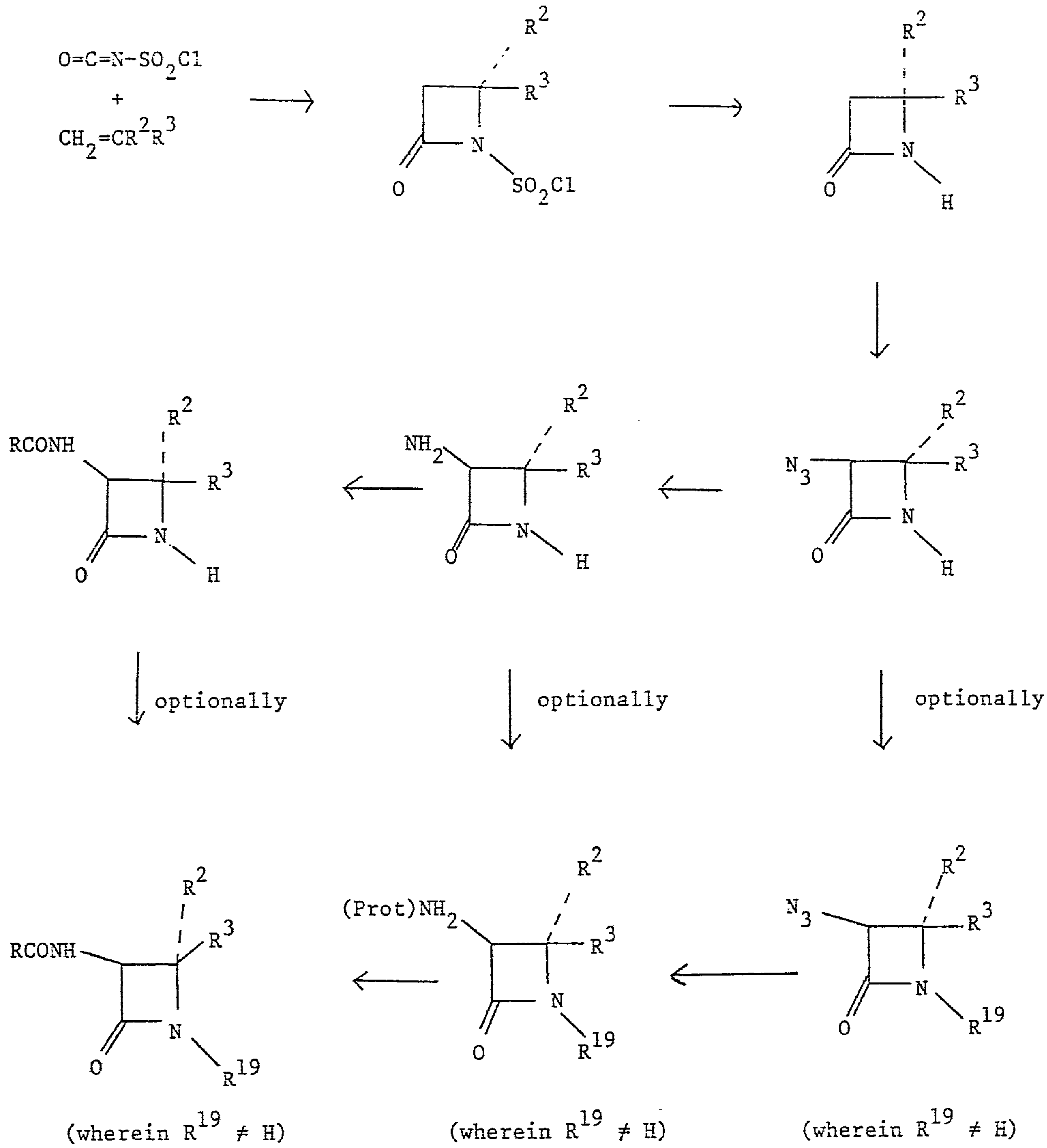


(Prot = optionally protected)

SCHEME 2

Applicable when $R^2$ and $R^3$ are each hydrogen.

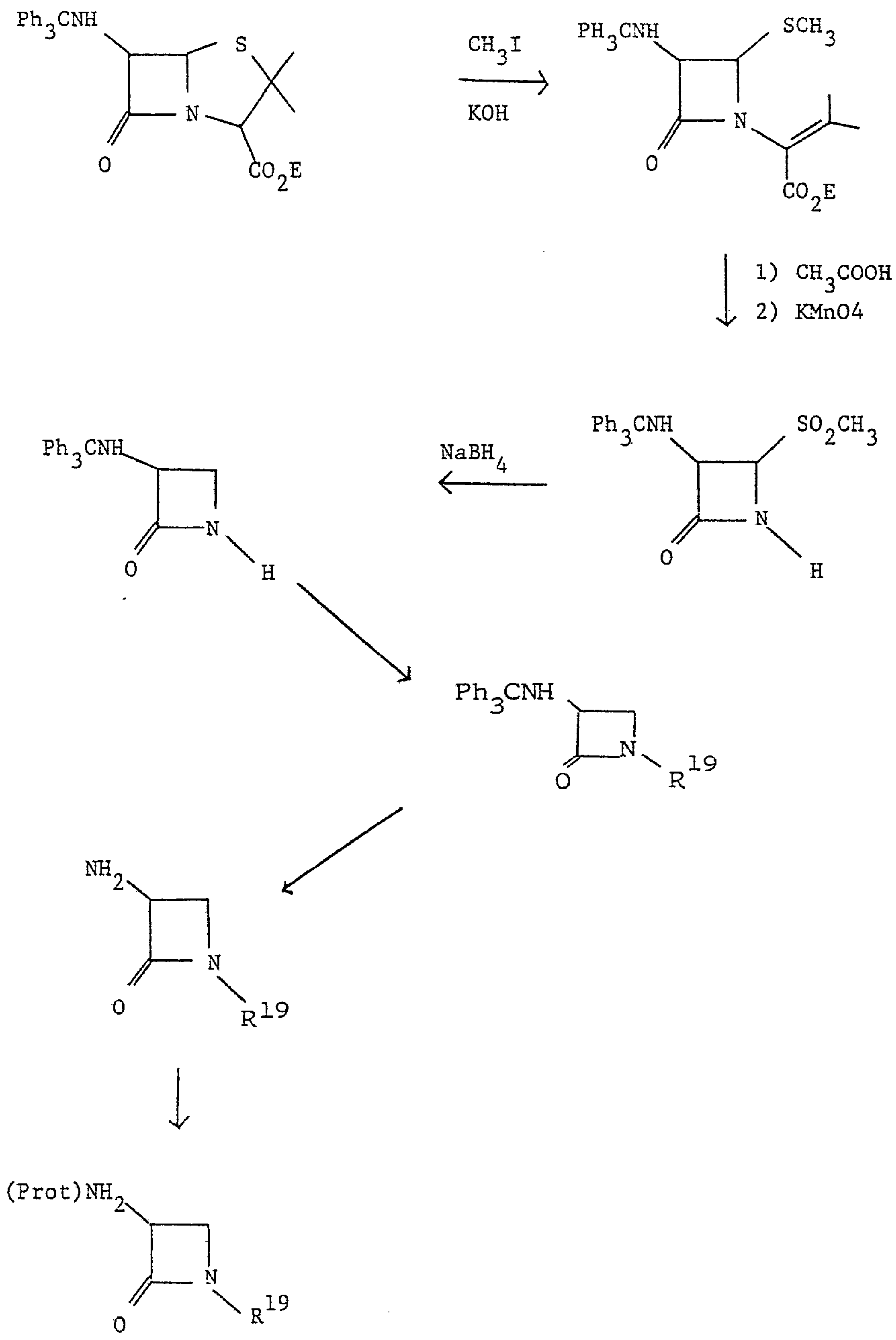


(wherein $R^{19} \neq H$)

0086563

SCHEME 3

Applicable when at least one of $R^2$ and $R^3$ is hydrogen.

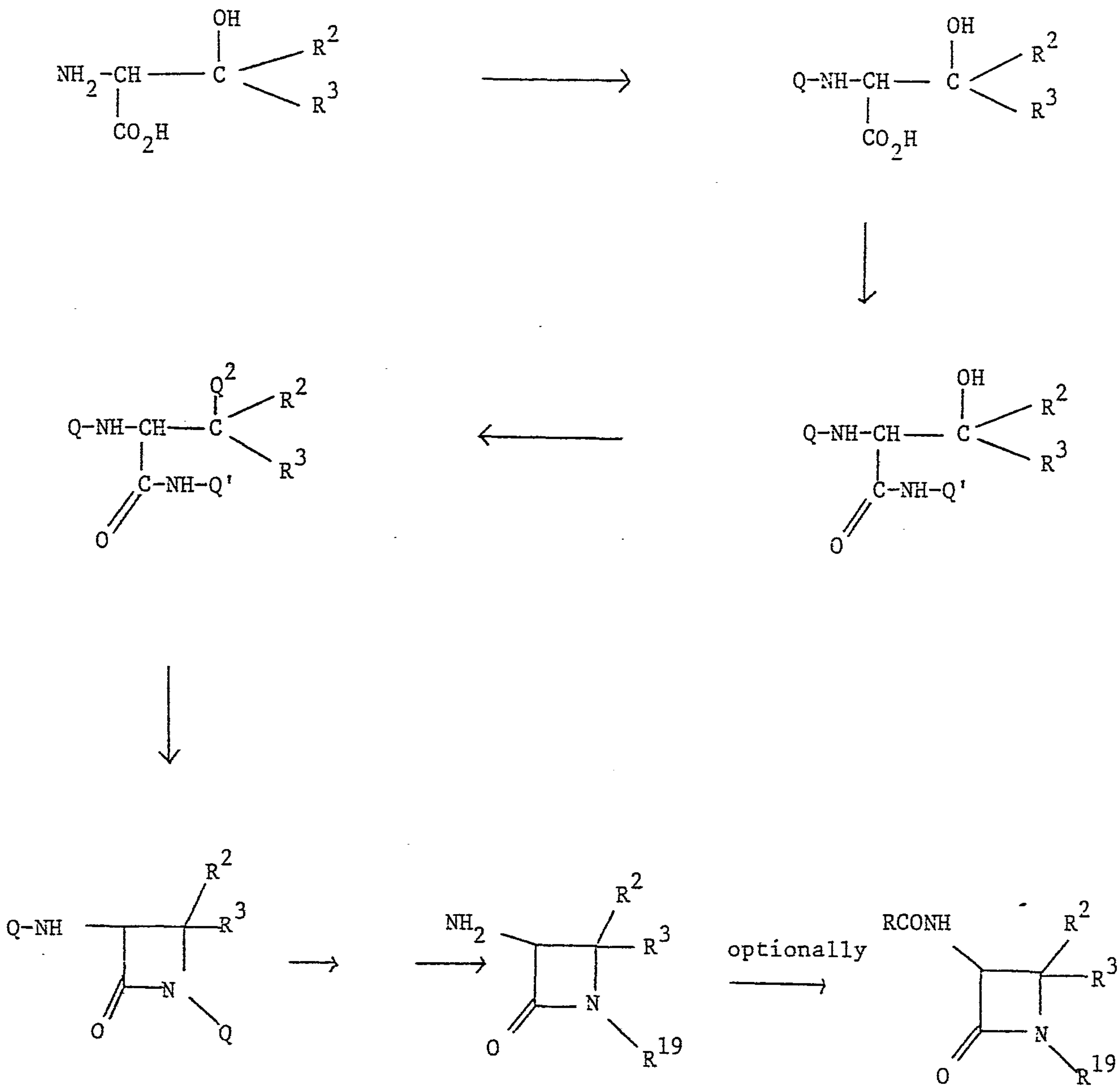


Q and Q' are protecting groups

$Q^2$ is a leaving group

A preferred variant of Scheme 3 is that where the nitrogen atom is protected as a phthalimido group (that is Q-NH- is replaced by phthalidmido). This prevents by-products arising from intramolecular cyclization of the side-chain -NH- moiety.

For convenience we have only depicted one enantiomer of the monocyclic β-lactams described herein (with the exception of compounds ()-() in the examples hereinafter). These enantiomers are believed to be the more active enantiomers. Resolution may be performed using conventional methods at any appropriate stage of the synthetic sequence.

Example 1

(DL)-2-Benzyloxycarbonylamino-3-hydroxy-N-p-methoxymethoxyphenyl propionamide (1)

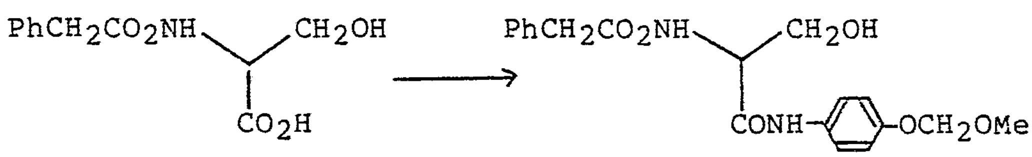

(1)

(DL)-Benzyloxycarbonylserine (42.67 g) was dissolved in dry tetrahydrofuran (300 ml) and p-methoxymethoxyaniline (27.54 g) added. The mixture was cooled to 0°C and treated with dicyclohexylcarbodiimide (40.79 g) in dichloromethane (100 ml). The solution was warmed to room temperature and after 30 min the solid was removed and the filtrate evaporated. Trituration of the residue with ether gave a white solid (1)(59.17 g) slightly contaminated with dicyclohexylurea, but sufficiently pure for the next stage of the process;

$\nu_{max}$ (Nujol) 3425, 3280, 1700, 1675 and 1660 $cm^{-1}$;

$\delta$($CDCl_3$) 3.38 (3H, s), 3.75 br (1H, s, becomes d, *J* 5.5Hz on $D_2O$ exch.), 4.34 (1H, m, becomes t, *J* 5.5Hz on $D_2O$ exch.), 4.76 br (1H, s, exch. $D_2O$), 5.07 (2H, s), 6.76 (1H, d, J 9Hz, exch. $D_2O$), 6.9 (2H, d, *J* 8Hz), 7.3 (5H, s), 7.49 (1H, d, *J* 8Hz) and 9.48 (1H, s, exch. $D_2O$).

Example 2

[3RS]-3-Benzyloxycarbonylamino-1-(p-methoxymethoxyphenyl)-azetidin-2-one (2)

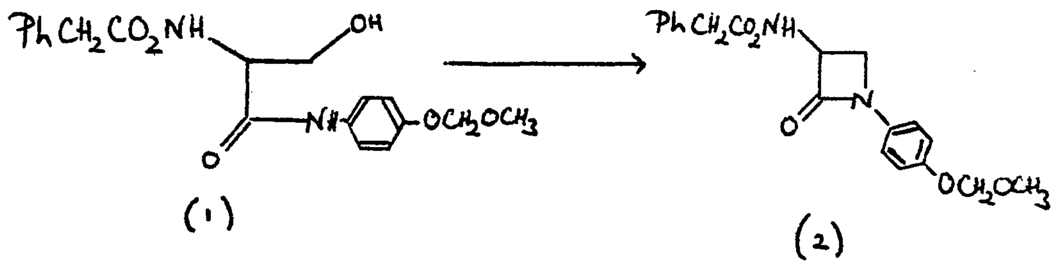

(1)

(2)

The amide (1) (4.94 g) was dissolved in dry tetrahydrofuran (150 ml) and the solution cooled to 0°C. Triphenylphosphine (3.8 g) was added, followed by the dropwise addition of diethyl azodicarboxylate (2.53 g) in tetrahydrofuran (5 ml). The solution was warmed to room temperature and after 15 min poured into ethyl acetate-water. The organic layer was separated, washed with brine, dried and evaporated. Chromatography on silica using ethyl acetate/methylene dichloride mixtures gave material which on recrystallisation from ethyl acetate-hexane provided the βlactam (2)(1.75 g), mp 153-154°C;

$\nu_{max}$ ($CHCl_3$) 3430, 1750 and 1730 $cm^{-1}$;

$\delta$($CDCl_3$) 3.44 (2H, s), 3.55 (1H, dd, J 5.5 and 3Hz), 3.88 (1H, dd), 4.91 (1H, m), 5.55 (1H, m) and 6.9-7.5 (10H, m).

Example 3

(3RS)-3-Amino-1-(p-methoxymethoxyphenyl)azetidin-2-one (3)

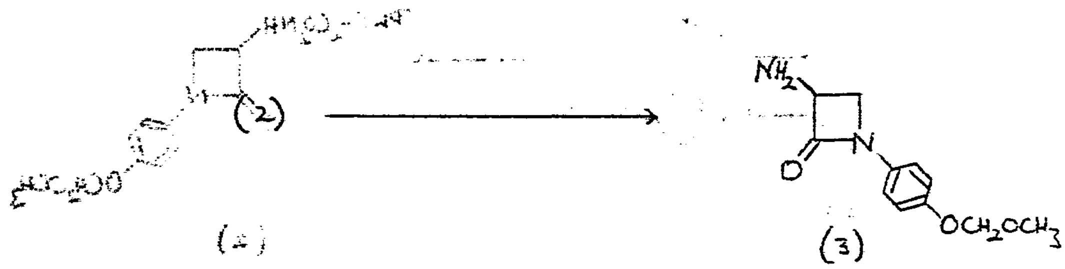

(3)

The lactam (2) (3.2 g) was dissolved in dry dioxan (100 ml) and hydrogenated over 10% palladium charcoal (1.0 g) until the uptake of hydrogen ceased. The mixture was filtered through Kieselguhr and the filtrate was evaporated to give a white solid (3) (1.95 g);

$\nu_{max}$ ($CHCl_3$) 3365, 1738 $cm^{-1}$;

$\delta$($CDCl_3$) 1.74 (2H, s, exch. $D_2O$), 3.31 (1H, d, *J* 6.5 and 3Hz), 3.44 (2H, s), 3.88 (1H, dd, *J* 6.5 and 6Hz), 4.3 (1H, dd, *J* 6 and 3Hz), 5.11 (2H, s), 6.98 (2H, d, J 9Hz) and 7.17 (2H, d, *J* 9Hz).

Example 4

(3RS)-1-(p-Methoxymethoxyphenyl)-3-phthalimido-azetidin-2-one (6)

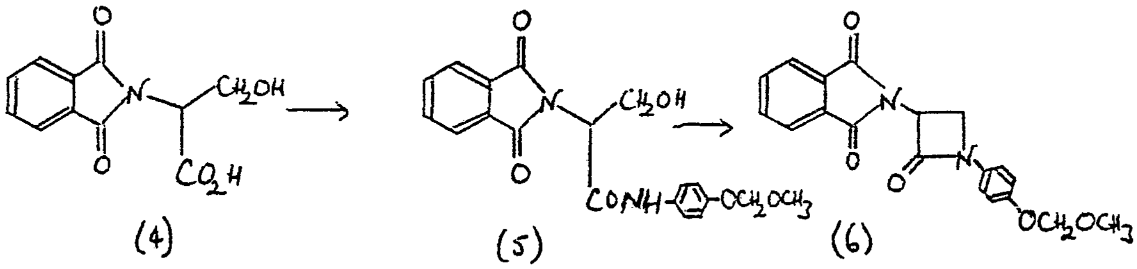

(4) (5) (6)

N-Phthaloylserine (4)(13.38 g; ca 80% pure by nmr) and p-methoxymethoxyaniline (6.96 g) were dissolved in dichloromethane (120 ml) and tetrahydrofuran (10 ml). The solution was then cooled to 0°C and treated with dicyclohexylcarbodi-imide in dichloromethane (20 ml). The reaction mixture was allowed to warm to room temperature and after 1.5h the solid was removed and the filtrate evaporated. The crude product (5) was sufficiently pure for the next step, and was dissolved in dry tetrahydrofuran (300 ml) at 0°C. Triphenylphosphine (17.88 g) was added followed by the dropwise addition of diethyl azodicarboxylate (11.65 g) in tetrahydrofuran (30 ml). The reaction mixture was allowed to warm to room temperature and after 0.5h the solvent was evaporated off and the residue chromatographed on silica to provide (6) as a crystalline solid (12 g), m.p. 156°C; $\nu_{max}$ (Nujol), 1790 (weak), 1740, 1720 $cm^{-1}$; ($CDCl_3$) 3.45 (3H, s), 4.0 (2H, d, J 5Hz), 5.13 (2H, s), 5.46 (1H, t, J 5Hz), 7.01 (2H, d, J 9Hz), 7.35 (2H, d, J 9Hz), and 7.65-7.95 (4H, m). (Found: C, 64.7; H, 4.5; N, 7.9. $C_{19}H_{16}N_2O_5$ requires C, 64.8; H, 4.5; N, 8.0%).

Example 5

(3RS)-3-Amino-1-(p-methoxymethoxyphenyl)azetidin-2-one (3)

(6) 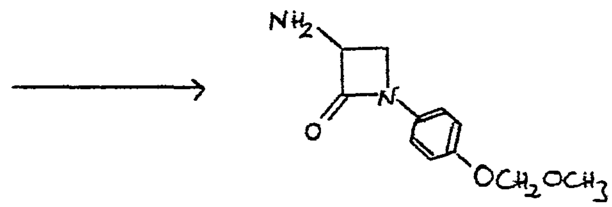

(3)

The lactam (6) (7.04 g) was dissolved in chloroform (80 ml) and N-methylhydrazine (2.02 g) added. The solution was stored at room temperature in the dark for 66 hours. The precipitated solid was filtered off. The filtrate was evaporated and the residue triturated with ether to provide (3) (4.04 g), identical to that described in Example 3.

Example 6

(3RS)-1-pMethoxymethoxyphenyl-3-N-p-nitrobenzylidene-amino-azetidin-2-one (7)

(3) 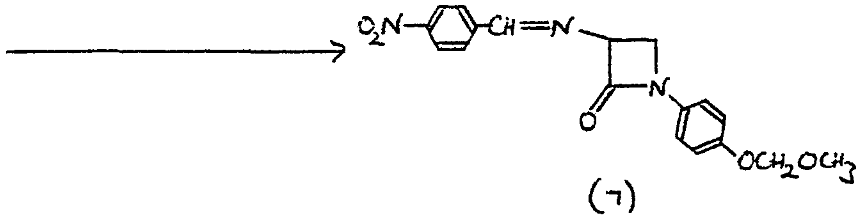

(7)

A solution of the amine (3) (2.64 g) and p-nitrobenzaldehyde (1.80 g) in an anhydrous mixture of toluene (100 ml) and dichloromethane (20ml) was stirred for 20 hours at room temperature over 4A molecular sieves. The reaction mixture was then filtered and the filtrate evaporated to dryness. The residue was recrystallised from ethyl acetate-hexane to afford the product (7) as pale yellow needles (3.7 g). m.p. 139-140° $\nu_{max}$ (Nujol) 1740, 1630, 1515 and 1350 $cm^{-1}$; $\delta$ ppm ($CDCl_3$) 3.46 (3H, s), 3.86 and 4.07 (2H, ABq, *J* 5.5Hz, higher field arm further coupled, d, *J* 2.1Hz, lower field arm further coupled, d, *J* 5.4 Hz), 5.03 (1H, dd, *J* 5.4 and 2.1Hz), 5.14 (2H, s), 7.03 and 7.33 (4H, ABq, *J* 10Hz), 7.93 and 8.26 (4H, d, *J* 9Hz), 8.62 (1H, s); (Found: C, 60.9; H, 4.9; N, 11.9; $C_{18}H_{17}N_3O_5$ requires C, 60.8; H, 4.8; N, 11.8%).

Example 7

(3RS)-3-Hydroxymethyl-1-p-methoxymethoxyphenyl-3-N-p-nitrobenzylideneamino-azetidin-2-one (8)

(7)

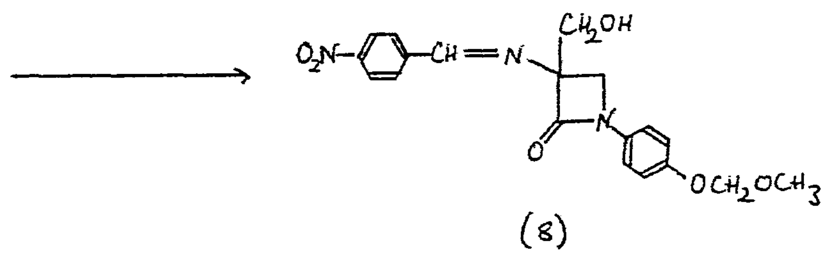

(8)

Anhydrous potassium carbonate (0.867 g) was added to a solution of the azetidinone (7) (2.23 g) in dry dimethylformamide (20 ml), cooled to 0°C under argon. After 10 mins anhydrous paraformaldehyde (1.00 g) was heated to 170°C under a stream of argon and the resulting gaseous mixture was passed over the above stirred solution. After completion of the depolymerisation of the paraformaldehyde, the reaction mixture was allowed to warm to ambient temperature and stirred for 2 hours. It was then diluted with ethyl acetate and water, filtered through Kieselguhr and the aqueous phase separated. The organic phase was then washed with water (x5), brine (x2), dried ($MgSO_4$), filtered and evaporated. The residual solid was recrystallised from ethyl acetate-hexane to afford the product (8) (2.29 g) m.p. 154-155°; $\nu_{max}$ (Nujol) 3475, 1725, 1630, 1515, 1345 $cm^{-1}$; $\delta$ ppm ($CDCl_3$) 2.51 (1H, t, J 6Hz, exchange), 3.44 (3H, s), 3.73 and 4.07 (2H, ABq, J 6Hz), 4.08 (2H, d, J 6Hz, collapsing to s, on exchange), 5.13 (2H, s), 7.00 and 732 (4H, ABq, J 9Hz), 7.93 and 8.25 (4H, ABq, J 8Hz), 8.86 (1H, s). (Found: C, 59.5; H, 5.1; N, 10.6, $C_{19}H_{19}N_3O_6$ requires C, 59.2; H, 4.9, N, 10.9%).

Example 8

(3RS)-3-Amino-3-hydroxymethyl-1-p-methoxymethoxyphenyl-azetidin-2-one-p-toluenesulphonic acid salt (9)

(8) 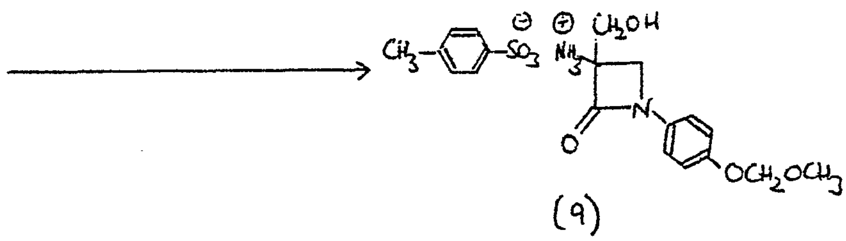

(9)

The azetidinone (8) (2.29 g) was dissolved in ethyl acetate (200 ml) and a solution of p-toluene-sulphonic acid monohydrate (1.13 g) in ethyl acetate (10ml) added over 1-2 mins, at room temperature. Precipitation occurred almost immediately and after stirring for 1.5 hours the product was filtered off, washed well with ethyl acetate and dried *in vacuo* over $P_2O_5$ to afford the tosylate salt (9) as a white solid (2.32 g). $\nu_{max}$ (Nujol) 3200br, 1750 $cm^{-1}$; $\delta$ ppm [$(CD_3)_2SO$] 2.27 (3H, s), 3.35 (3H, s), 3.6-4.1 (4H, m), 5.15 (2H, s), 6.9-7.6 (8H, m), 8.6-9.3br (exchange $D_2O$).

Example 9

(3RS)-3-Amino-3-hydroxymethyl-1-p-methoxymethoxyphenyl-azetidin-2-one (10)

(9)

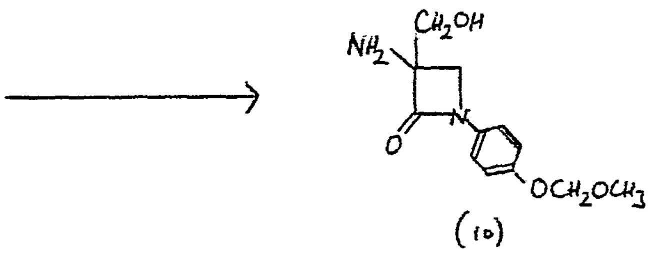

(10)

The tosylate (9) (0.02 g) was suspended in dichloromethane (3 ml) and the minimum volume of ammonium hydroxide required to give two separable phases added. The organic phase was separated, dried ($MgSO_4$) and evaporated to give the product (10) as an oil (0.006 g) $\nu_{max}$ ($CHCl_3$) 3370, 1740 $cm^{-1}$; $\delta$ ppm ($CDCl_3$) 2.24 br (3H, s, exchange), 3.48 (3H, s), 3.74 and 3.83 (2H, ABq, J 5.87Hz), 3.85 (2H, s), 5.15 (2H, s), 7.00-7.06 (2H, m), 7.25-7.33 (2H, m). (Found: $M^+$ 252.1105, $C_{12}H_{16}N_2O_4$ required M, 252.1110).

Example 10

(3-RS)-3-[D,-2-(4-Ethyl-2,3-dioxopiperazin-1-yl-carboxamido)-2-phenylacetamido]-3-hydroxymethyl-1-p-methoxymethoxyphenyl-azetidin-2-one (11)

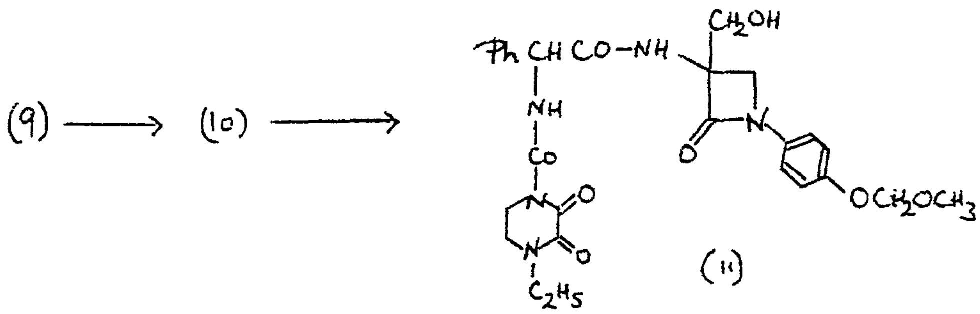

(9) (10) (11)

The tosylate (9) (1.696 g) was suspended in anhydrous dichloromethane (80 ml) and triethylamine (0.62 ml) added dropwise over 1-2 mins at 0°C, followed by finely ground 4A molecular sieves (5.9 g). To the vigorously stirred mixture was added freshly prepared D-2-(4-ethyl-2,3-dioxopiperazin-1-yl-carboxamido)-2-phenylacetyl chloride (1.49 g) in dry dichloromethane (20 ml) dropwise over 5-10 mins. After the addition, the reaction mixture was allowed to reach room temperature, stirred for 2 hours and then filtered. The filtrate was washed with dilute hydrochloric acid, aqueous sodium hydrogencarbonate, brine, dried ($MgSO_4$) and evaporated. The residue was chromatographed on silica gel to afford the azetidinone (11) as a white amorphous solid (0.64 g) $\nu_{max}$ ($CHCl_3$) 3400, 3300, 1740, 1720, 1690 $cm^{-1}$; $\delta$ ppm ($CDCl_3$) 1.13 (1H, t, *J* 7Hz), 1.19 (1H, s, exchange) 3.42 (3H, s), 3.3-4.2 (10H, complex m), 5.09 (2H, s), 5.51 (1H, d, *J* 7Hz), 6.9-7.7 (9H, m), 8.04 (1H, s), 9.78 (1H, d, *J* 7Hz).

Example 11

(3RS)-3-tert-Butyldimethylsilyloxymethyl-3-[D-2-(4-ethyl-2,3-dioxopiperazin-1-yl-carboxamido)-2-phenylacetamido]-1-p-methoxymethoxyphenyl-azetidin-2-one (13)

Method 1

(11)

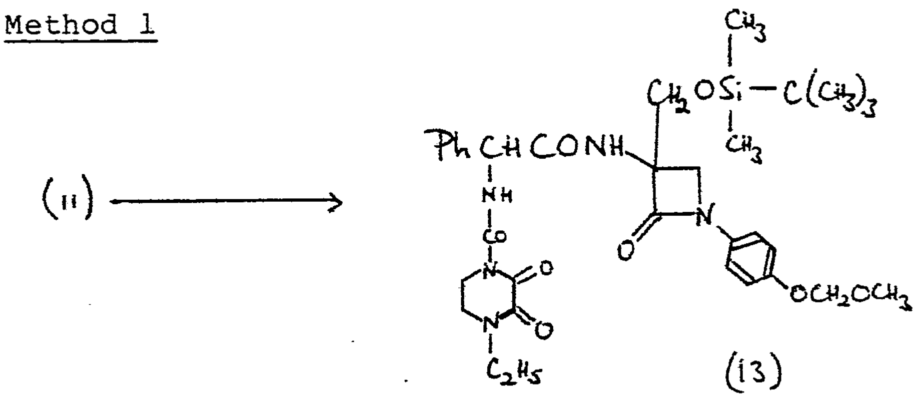

(13)

The β-lactam (11) (0.643 g) was dissolved in dry dimethylformamide (10 ml) containing t-butyldimethylsilyl chloride (0.21 g) and imidazole (0.174 g) at room temperature. After 19 hours the reaction mixture was diluted with ethyl acetate, washed with brine (x3), dried ($MgSO_4$) and evaporated. Chromatography of the residue on silica gel afforded the product (13) as an amorphous solid (0.698 g). $\nu_{max}$ ($CHCl_3$) 3400, 3275, 1742, 1718, 1685 $cm^{-1}$; $\delta$ ppm ($CDCl_3$) 0.04 (6H, s), 0.78 and 0.82 (9H, s), 1.23 (3H, t, J 7Hz), 3.4-3.6 (2H, m), 3.50 and 3.51 (3H, s), 3.59 (2H, q, J 7Hz), 3.65-4.20 (6H, m), 5.17 and 5.18 (2H, s), 5.48 (~0.7H, d, J 7Hz), 5.50 (~0.3H, d, J 7Hz) 6.57 (~0.3H, s), 6.66 (~0.7H, s), 6.95-7.05 (2H, m), 7.20-7.50 (7H, m), 9.94 (~0.7H, d, J 7Hz), 9.97 (~0.3H, d, J 7Hz).

Method 2

(9)

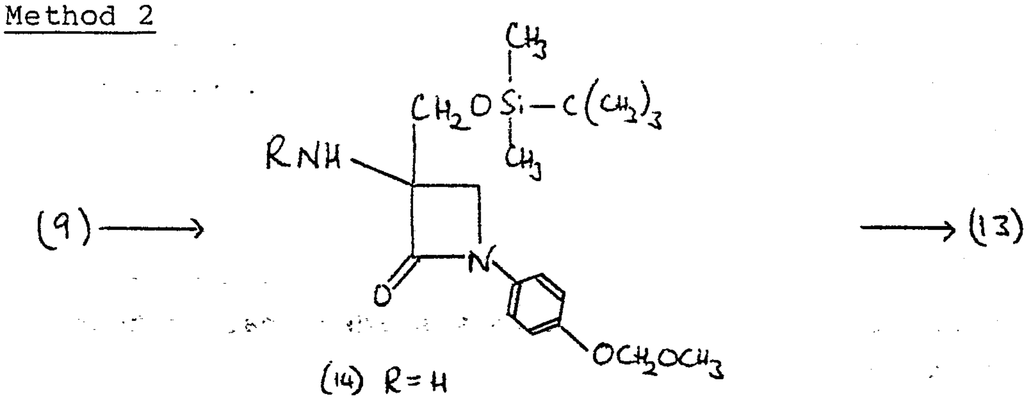

(13)

(14) R = H
(15) R = COH

The tosylate (9) (0.575 g) was dissolved in dry dimethylformamide (10 ml) containing t-butyldimethylsilyl chloride (0.235 g) and imidazole (323 mg) at room temperature. After 19 hours the solvent was evaporated and reevaporated from toluene. The process was repeated to give the crude amine (14) as an oil which was dried in vacuo for 40 mins.

The total product from above was dissolved in dry dichloromethane (25 ml) at 0°C and vigorously stirred with finely ground 4A molecular sieves (3g) whilst freshly prepared D-α-(4-ethyl-2,3-dioxopiperazin-1-ylcarboxamido)-phenylacetyl chloride (0.686 g) in dry dichloromethane (10 ml) was added dropwise over 5 mins. The reaction mixture was allowed to reach room temperature, stirred for 3 hours and filtered. The filtrate was washed with dilute hydrochloric acid, aqueous sodium hydrogencarbonate, brine, dried ($MgSO_4$) and evaporated. Chromatography of the residue on silica gel afforded two major β-lactam products. The least polar (t.l.c) product was (3RS)-3-tert-Butyldimethylsilyloxymethyl-3-formamido-1-p-methoxymethoxyphenylazetidin-2-one (15) isolated as an oil (0.111 g) $\nu_{max}$ ($CHCl_3$) 3400, 1750, 1690 $cm^{-1}$; δ ppm ($CDCl_3$) 0.00 and 0.02 (6H, s), 0.82 (9H, s), 3.43 (3H, s), 3.82

and 4.06 (2H, ABq, J 10Hz), 3.86 and 3.96 (2H, ABq, J ~ 5Hz), 5.12 (2H, s), 6.69 (1H, broad s, exchange), 6.9-7.4 (4H, m), 8.12 (1H, d, J ~1.5Hz, collapses to s, on exchange). (Found $M^+$ 394.1911, $C_{19}H_{30}N_2O_5Si$ requires M, 394.1924).

Further elution of the column gave the required product (13) (0.22 g) which was identical to that described in Method 1.

Example 12

(3RS)-3-tert-Butyldimethylsilyloxymethyl-3[D-2-(4-ethyl-2,3-dioxopiperazin-1-yl-carboxamido)-2-phenyl-acetamido]-azetidin-2-one (16)

(13)

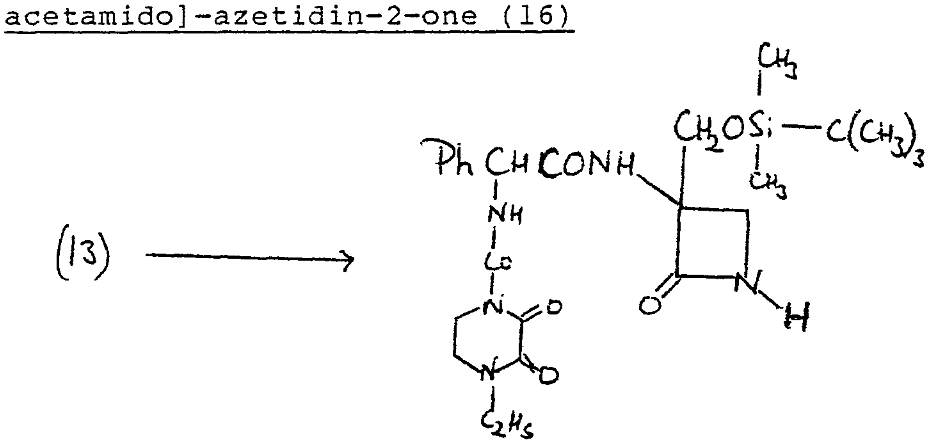

The β-lactam (13) (0.144 g) in tetrahydrofuran (3ml) was cooled to 0°C and ceric ammonium nitrate (CAN) (0.591 g) in water (1.5 ml) added dropwise over 2-3 mins to the vigorously stirred solution. (T Fukuyama, L K Frank and C F Jewell, Jr., J Amer Chem Soc., 1980, 102, 2122). After 10 mins solid sodium sulphite was added to decolourise the mixture, which was then poured into dichloromethane and dilute sodium hydrogencarbonate. The mixture was filtered, the organic phase separated and washed with brine, dried ($MgSO_4$) and evaporated. Chromatography of the residue on silica afforded the products (16) as a separable mixture of diastereoisomers (0.054 g).

Diastereoisomer 1 : $\nu_{max}$ ($CHCl_3$) 3410, 3275, 1770, 1715, 1685 $cm^{-1}$; δ ppm ($CDCl_3$) 0.2 and 0.3 (6H, s), 0.85 (9H, s), 1.19 (3H, t, J ~ 7Hz), 3.4-3.6 (6H, m), 3.75 and 3.91 (2H, ABq, J 10.17Hz), 3.95-4.10 (2H, m), 5.47 (1H, d, J 7.02Hz), 6.24 (1H, s), 6.90 (1H, s), 7.3-7.5 (5H, m), 9.90 (1H, d, J 7.02Hz).

Diastereoisomer 2 : $\nu_{max}$ ($CHCl_3$) 3420, 3270, 1775, 1718, 1690 $cm^{-1}$; $\delta$ ppm ($CDCl_3$) 0.0 and 0.2 (6H, s), 0.8 (9H, s), 1.22 (3H, t, J ~ 7Hz), 3.35 and 3.65 (6H, m), 3.71 and 3.90 (2H, ABq, J 10.17Hz), 3.9-4.2 (2H, m), 5.49 (1H, d, J 5.83Hz), 6.15 (1H, s), 6.6 (1H, s), 7.3-7.45 (5H, m), 9.96 (1H, d, J 5.83Hz).

Example 13

(3RS)-DiPotassium salt of 3-[D-2-(4-Ethyl-2,3-dioxo-piperazin-1-yl-carboxamido)-2-phenylacetamido]-3-hydroxysulphonyloxymethyl-2-oxo-azetidin-1-sulphonic acid (7) and

(3RS)-Potassium 3-[D-2-(4-Ethyl-2,3-dioxopiperazin-1-yl-carboxamido)-2-phenylacetamido]-3-hydroxymethyl-2-oxo-azetidin-1-sulphonate (18)

(16) 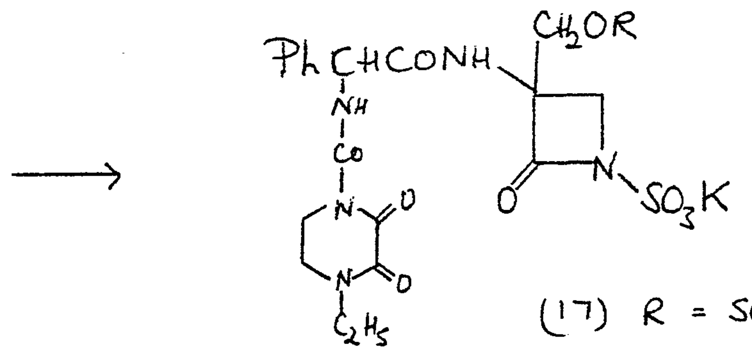

(17) R = $SO_3K$

(18) R = H

The azetidinone (16) (0.053 g) was dissolved in dry dimethylformamide (0.5 ml), pyridine-sulphur trioxide complex (0.032 g) added and the solution kept at room temperature under argon for 5 days. Trituration with ether gave an oily precipitate which was passed down Amberlite IR-120 resin ($K^+$ form) and chromatographed on HP20-SS to give the product (17) (~2:1 mixture of isomers) as a white solid (0.015g). $\nu_{max}$ (KBr) 3460, 1765, 1710, 1675, 1510, 1250, 1185, 1055, 1010 $cm^{-1}$; $\delta$ ppm ($D_2O$) 1.19 (3H, t, J 7Hz), 3.51 (2H, q, J 7Hz), 3.6-3.9 (~2 1/3H, m), 3.80 and 3.94 (~1 1/3H, ABq, J ~5Hz), 3.86 (~1/3H, lower field arm of ABq, J ~ 5Hz, higher field arm obscured by m, 3.6-3.9) 3.95-4.15 (2H, m), 5.31 and 5.44 (~ 1 1/3H, ABq, J ~ 12Hz), 5.38 (~ 2/3H, AA'), 5.49 (~ 2/3H, s), 5.52 (~ 1/3H, s), 7.48 (5H, s), 7.93 (1H, s).

Further elution of the column gave (18) (~2:1 mixture of diastereoisomers at C-3) as a white solid (4 mg). $\nu_{max}$ (KBr) 3450, 1763, 1710, 1670, 1512, 1250, 1190, 1050 $cm^{-1}$; $\delta$ ppm ($CDCl_3$) 1.19 (3H, t, J 7Hz), 3.51 (2H, q, J 7Hz), 3.55-4.10 (8H, m), 5.50 (~2/3H, s), 5.53 (~1/3H, s), 7.48 (5H, s).

Example 14

(3RS)-tert-Butyldimethylsilyloxymethyl-3-N-p-nitrobenzylideneamino-1-p-methoxymethoxyphenyl-azetidin-2-one (19)

(8)

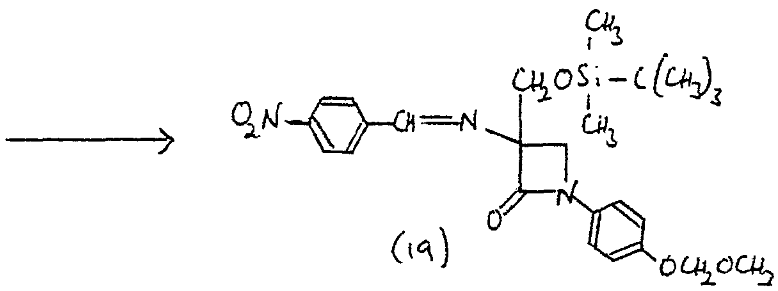

(19)

The β-lactam (8) (0.077 g) was dissolved in dimethylformamide (0.5 ml) containing imidazole (0.030g) and t-butyldimethylsilyl chloride (0.033 g) at room temperature. After 17 hours the solution was evaporated and then re-evaporated from toluene. This process was repeated. The residue was chromatographed on silica gel to afford the product (19) as a solid (0.095 g). $\nu_{max}$ ($CHCl_3$) 1740, 1630, 1520, 1345 $cm^{-1}$; $\delta$ ppm ($CDCl_3$) 0.13 (6H, s), 0.81 (9H, s), 3.43 (3H, s), 3.69 and 4.09 (2H, ABq, J 5Hz), 3.97 (2H, slightly broadened s), 5.12 (2H, s), 6.99 and 7.32 (2H, ABq, J 9Hz), 7.92 and 8.25 (2H, ABq, J 8Hz), 8.84 (1H, s). (Found : $M^+$ 499.2112, $C_{25}H_{33}N_3O_6Si$ requires M, 499.2146).

Example 15

(3RS)-3-Amino-3-tert-butyldimethylsilyloxymethyl-1-p-methoxymethoxyphenyl-azetidin-2-one-p-toluenesulphonic acid salt (20)

(19)

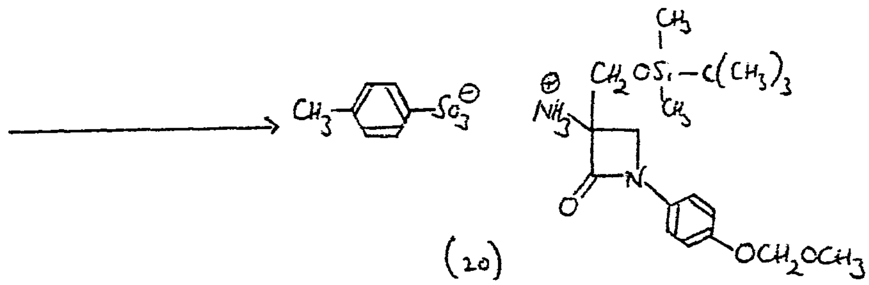

(20)

The β-lactam (19) (0.035 g) was dissolved in ethyl acetate (0.5 ml) and a solution of p-toluenesulphonic acid monohydrate (0.014 g) added as described in Example 8. The product (20) was isolated as a white solid (0.03 g) $\nu_{max}$ (Nujol) 3000 br, 1755 $cm^{-1}$; $\delta$ ppm $[(CD_3)SO]$ *inter alia* 0.7 (9H, s), 2.23 (3H, s), 3.30 (3H, s), 3.4-4.1 (4H, m), 5.1 (2H, s), 6.8-7.6 (8H, m).

Example 16

(3RS)-3-Benzyloxycarbonyloxymethyl-3-N-p-nitrobenzylidene-amino-1-p-methoxymethoxyphenyl-azetidin-2-one (21)

(8) 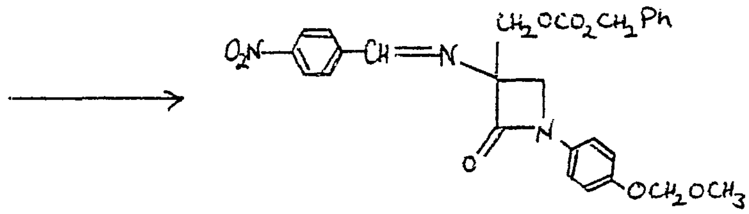

(21)

The β-lactam (8) (2.603 g) was dissolved in dry dichloromethane (50 ml) containing 4-N,N-dimethylaminopyridine (1.65 g) under argon, cooled to 0°C and benzyl chloroformate (90-95%; 1.20 ml) in dichloromethane (5ml) added dropwise over 10 mins. After 1 hour at 0-10°C, the reaction was allowed to reach room temperature over 1 hour, diluted with dichloromethane and washed with brine. The organic phase was separated dried ($MgSO_4$) and evaporated. Chromatography on silica gel afforded the product (21) as a pale yellow solid (2.98 g). $\nu_{max}$ ($CHCl_3$) 1750, 1640, 1515, 1350 $cm^{-1}$; $\delta$ ppm ($CDCl_3$) 3.48 (3H, s), 3.81 and 4.08 (2H, ABq, *J* ~6.5Hz), 4.57 and 4.68 (2H, ABq, *J* ~ 11.5Hz), 5.16 (4H, s), 7.04 and 7.33 (4H, ABq, *J* ~ 10Hz), 7.35 (5H, s), 7.93 and 8.26 (4H, ABq, *J* ~ 8Hz), 9.88 (1H, s). (Found : $M^+$, 519.1625, $C_{27}H_{25}H_3O_8$ requires *M*, 519.1640).

Example 17

(3RS)-3-Amino-3-Benzyloxycarbonyloxymethyl-1-p-methoxy-methoxyphenyl-azetidin-2-one p-toluene sulphonic acid salt (22)

(21)

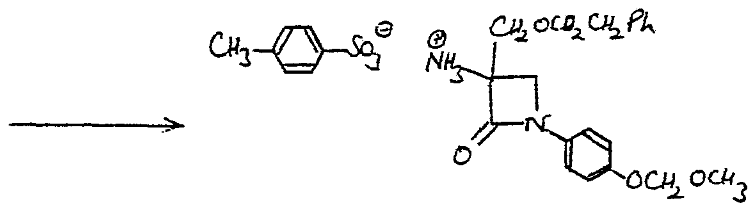

(22)

The azetidinone (21) (0.52 g) was dissolved in dichloromethane (6 ml) and ethyl acetate (4 ml) and p-toluenesulphonic acid monohydrate (0.197 g) in a little ethyl acetate added as described in Example 8. The product (22) was isolated as a white solid (0.52g). $\nu_{max}$ (Nujol) 1750, 1630 $cm^{-1}$; $\delta$ ppm [$(CD_3)_2SO$] 2.29 (3H, s), 3.37 (3H, s), 3.81 and 3.99 (2H, ABq, J 7.05Hz), 4.64 (2H, AA'), 5.18 (2H, s), 5.20 (2H, s), 7.05-7.15 (4H, m), 7.3-7.5 (9H, m), 9.10 (3H, broad s).

Example 18

(3RS)-3-Benzyloxycarbonyloxymethyl-3-[D-2-(4-ethyl-2,3-dioxopiperazin-1-yl-carboxamido)-2-phenylacetamido]-1-p-methoxymethoxyphenyl-azetidin-2-one (24)

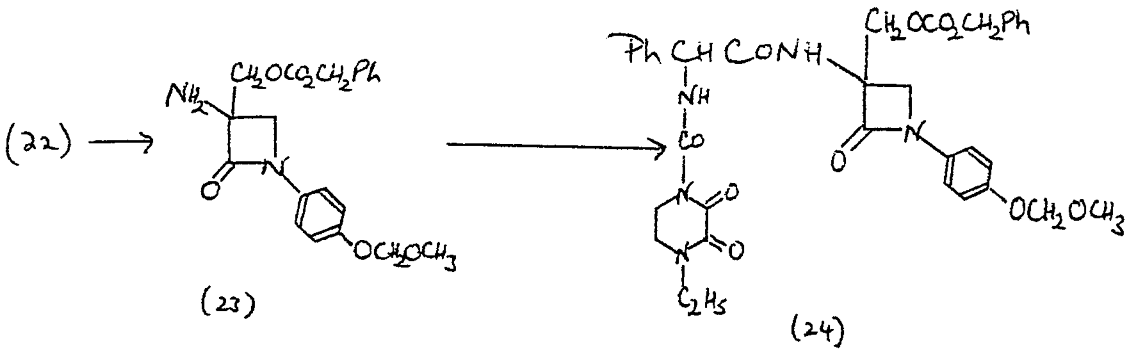

(22) (23) (24)

The tosylate (22) (0.5 g) was suspended in anhydrous dichloromethane (15 ml) and triethylamine (0.14 ml) added at 0°C. To the resulting solution of amine (23) was added pyridine (0.08 ml) followed by freshly prepared D-2-(4-ethyl-2,3-dioxopiperazin-1-yl-carboxamido)-2-phenylacetyl chloride (0.34 g) in dry dichlormethane (5 ml) as described in Example 10. The product (24) (~1:1 mixture of diastereoisomers at C-3) was isolated as a white amorphous solid (0.48 g). $\nu_{max}$ ($CHCl_3$) 3300, 1755, 1720, 1690 $cm^{-1}$; $\delta$ ppm ($CDCl_3$) 1.19 (3H, t, J 7Hz), 3.45 and 3.46 (3H, s), 3.4-3.6 (4H, m), 3.77-4.2 (4H, m), 4.42 and 4.50 (~ 1H; ABq, J ~11Hz), 4.53 (~ 1H, s), 5.07 (~ 1H, s), 5.13 (2H, s), 5.14 (~1H, s), 5.49 (~ 1H, d, J ~ 7Hz), 5.50 (~1H, d, J ~ 7Hz), 6.9-7.5 (15H, m), 9.88 (~ ½H, d, J ~7Hz), 9.89 (~ ½H, d, J ~ 7Hz).

0086563

Example 19

(3RS)-3-Benzyloxycarbonyloxymethyl-3-[D-2-(4-ethyl-2,3-dioxopiperazin-1-yl-carboxamido)-2-phenylacetamido]-azetidin-2-one (25)

(24)

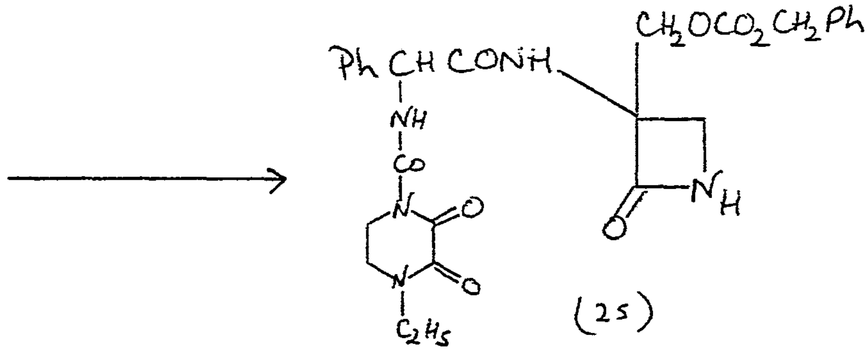

(25)

The β-lactam (24) (398 mg) in tetrahydrofuran (16ml) was cooled to 0°C and (CAN) (960 mg) in water (8 ml) added dropwise over 2-3 mins to the vigorously stirred solution as described in Example 12. The product (25) (~1:1 mixture of diastereoisomers at C-3) was isolated as a white amorphous solid (227 mg). ν max ($CHCl_3$) 3410, 3375, 1770, 1750, 1720, 1690 $cm^{-1}$; δ ppm ($CDCl_3$) 1.19 (3H, t, J ~ 7Hz), 3.4-3.7 (6H, m), 3.9-4.1 (2H, m), 4.34 and 4.46 (~ 1H, ABq, J ~ 11Hz), 4.48 (~ 1H, s), 5.07 (~ 1H, s), 5.10 (~ 1H, s), 5.49 (1H, d, J 7Hz), 6.21 (~ 1H, s, exchange), 6.42 (~ 1H, s, exchange), 7.2-7.45 (10H, m), 7.51 (~ ½H, s), 7.55 (~ ½H, s), 9.86 (1H, d, J ~ 7Hz).

Example 20

(3RS)-Potassium 3-Benzyloxycarbonyloxymethyl-3-[D-2-(4-ethyl-2,3-dioxopiperazin-1-yl-carboxamido)-2-phenylacetamido]-2-oxo-azetidin-1-sulphonate (26)

(25) 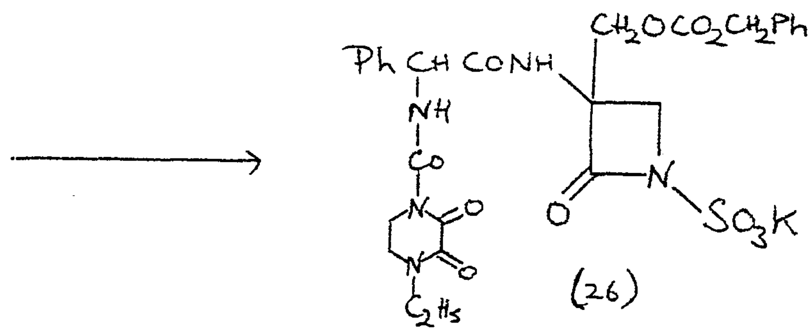

(26)

The azetidinone (25) (55 mg) was dissolved in dry dimethylformamide (0.5 ml), pyridinesulphur trioxide complex (32 mg) added and the solution kept at room temperature under argon for 4 days. The product (26) was isolated as described in Example 13. $\nu_{max}$ (KBr) 3450, 1765, 1710, 1675, 1510, 1270, 1180, 1050 $cm^{-1}$; $\delta$ ppm ($D_2O$) 1.16 (3H, t, J 7Hz), 3.47 (2H, q, J 7Hz), 3.55-4.0 (6H, m), 4.4-4.7 (2H, m), 5.11 and 5.13 (2H, two s), 5.43 and 5.45 (2H, two s), 7.45 (10H, s).

0086563

Example 21

(3RS)-3-Benzyloxycarbonyloxymethyl-3-[D-2-(4-ethyl-2,3-dioxopiperazin-1-yl-carboxamido)-2-phenylacetamido]-2-oxo-azetidin-1-sulphonic acid, Pyridinium salt (27)

(25)

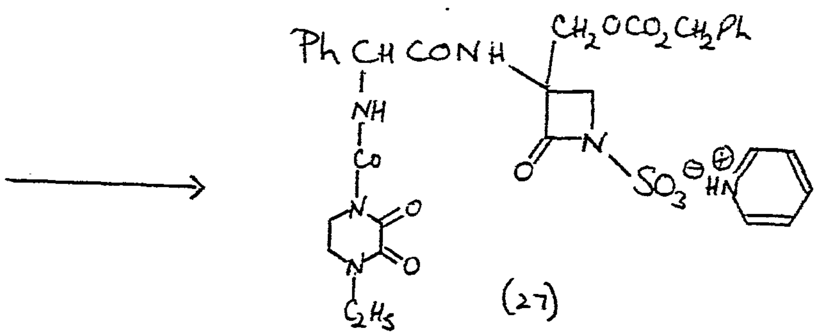

(27)

The azetidinone (25) (122 mg) was dissolved in dimethylformamide-sulphur trioxide complex (0.2 ml) at room temperature under argon. After 18 hours pyridine (19 µl) was added, and trituration with ether gave the required product (27) as a white solid (122 mg). $\nu_{max}$ (KBr) 3430, 1750, 1710, 1675, 1510, 1265, 1190, 1050 $cm^{-1}$.

Example 22

(3RS)-Potassium 3-[D-2-(4-Ethyl-2,3-dioxopiperazin-1-yl-carboxamido)-2-phenylacetamido]-3-hydroxymethyl-2-oxo-azetidin-1-sulphonate (18)

(27) ⟶ (18)

The β-lactam (27) (71 mg) was dissolved in dry dimethylformamide (4 ml) and hydrogenated over 10% palladium on charcoal catalyst (35 mg) for 12 mins. The reaction was filtered through Kieselguhr, the filtrate evaporated to low volume and triturated with ether to give an oily precipitate. Purification as described in Example 13 gave the required product (18) as a solid (13 mg). This material was identical in all respects to that described in Example 13.

Example 23

(3RS)-3-[D-2-(4-Ethyl-2,3-dioxopiperazin-1-yl-carboxamido)-2-phenylacetamido]-3-hydroxymethyl-azetidin-2-one (28)

(25)

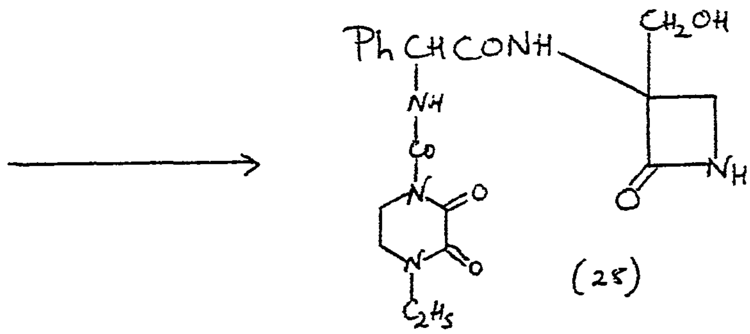

(28)

The azetidinone (25) (10 mg) was dissolved in dry dioxan (4 ml) and hydrogenated over 10% palladium on charcoal catalyst (5 mg) for 30 mins. The reaction was filtered through Kieselguhr, the filtrate evaporated and the residue chromatographed on silica gel to give the product (28) as a white solid (7 mg). $\nu_{max}$ ($CHCl_3$) 3300, 1760, 1715, 1685 $cm^{-1}$; $\delta$ ppm [$(CD_3)_2CO$] 1.13 (3H, t, *J* 7Hz), 3.35-3.85 (8H, m), 4.05 (2H, m), 5.68 ($\sim\frac{1}{2}$H, *J* 9Hz), 5.70 ($\sim\frac{1}{2}$H, *J* 9Hz), 7.09 ($\sim\frac{1}{2}$H, s, exchange), 7.12 ($\sim\frac{1}{2}$H, s, exchange), 7.3-7.6 (5H, m), 7.96 ($\sim\frac{1}{2}$H, s, exchange), 8.02 ($\sim\frac{1}{2}$H, s, exchange), 9.93 ($\sim\frac{1}{2}$H, d, *J* $\sim$9Hz), 9.98 ($\sim\frac{1}{2}$H, d, *J* $\sim$9Hz).

Example 24

(3S)-3-Triphenylmethylaminoazetidin-2-one (30)

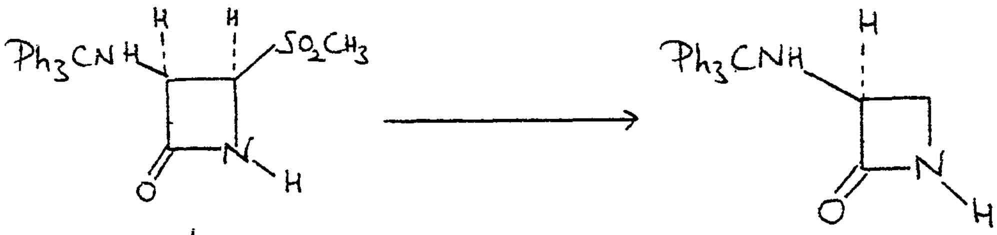

(29) (30)

The sulphone (29) (20.3g) (E G Brain et al. J C S Perkin Trans 1. 1976, p447) was dissolved in tetrahydrofuran (200 ml), cooled to 0°C under argon and sodium borohydride (3.8 g) in water (36 ml) added in four portions over 10 mins. The reaction was removed from the cooling bath, allowed to warm to room temperature over 1.5 hour and glacial acetic acid (5.72 ml) added. After 15 mins the solution was diluted with ethyl acetate (600 ml), washed with water (x2), saturated sodium hydrogencarbonate solution, brine, dried ($MgSO_4$) and evaporated. Chromatography on silica gel gave the product (30) as a white crystalline solid (14.3 g). m.p. 103-105° (toluene); $[\alpha]^{23}_D$ - 51.2° (c 1 $CHCl_3$); $\nu_{max}$ (Nujol) 3340, 3250, 1760, 1730 $cm^{-1}$; $\delta$ ppm ($CDCl_3$) 2.14 (1H, dd, *J* 5.9 + 2.4Hz), 2.53 (1H, d, *J* 10.8Hz, exchange), 2.63 (1H, dd, *J* 5.9 and 4.7Hz), 4.21 (1H, broad s, collapses to dd, *J* 4.7 and 2.4Hz on exchange), 5.53 (1H, broad s, exchange), 7.2 - 7.6 (15H, m). (Found: C, 81.3; H, 6.2; N, 7.8; $C_{22}H_{20}N_2O$. 1/3 $C_7H_8$ requires C, 81.5; H, 6.3; N, 7.8%).

Example 25

(3S)-1-t-Butyldimethylsilyl-3-triphenylmethylamino-azetidin-2-one (30)

(30)

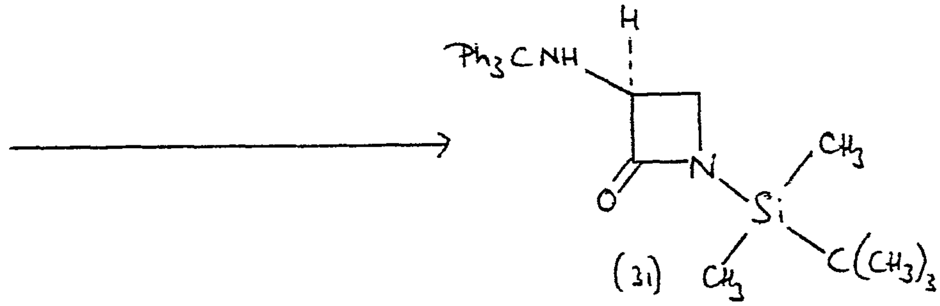

(31)

The azetidinone (30) (328 mg) was dissolved in dry dimethylformamide (3 ml) containing t-butyldimethylchlorosilane (166 mg) at 0°C and triethylamine (0.154 ml) in dry dimethylformamide (0.5 ml) added over 1 min. After 15 mins at 0°C and warming to room temprature, the reaction diluted with ethyl acetate, washed with water, very dilute hydrochloric acid, brine, dried ($MgSO_4$) and evaporated. Chromatography on silica gel afforded the product (31) as a white crystalline solid (338 mg). m.p. 166° (hexane); [ ]$^{19}_{D}$ - 56°; (c2 in $CHCl_3$) max (KBr) 3460, 1745 $cm^{-1}$; ppm ($CDCl_3$) 0.11 (6H, s), 0.86 (9H, s), 2.21 and 2.60 (2H, ABq, d, J 6Hz, higher field arm further coupled, d, J 2.5Hz, lower field arm further coupled, d, J 5Hz), 2.5-2.8 (1H, broad s, exchange), 4.20 (1H, m), 7.1-7.6 (15H, m). (Found: C, 76.1; H, 7.5; N, 6.3; $C_{28}H_{34}N_2OSi$ requires C, 76.0; H, 7.7; N, 6.3%).

Example 26

(3S)-3-Amino-1-t-butyldimethylsilyl azetidin-2-one (3 )

(31)

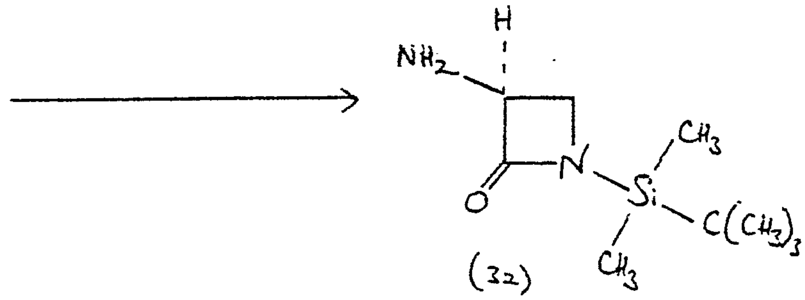

(32)

The β-lactam (31) (277 mg) was dissolved in anhydrous dichloromethane (4 ml), cooled to 0°C and p-toluene- sulphonic acid monohydrate (119 mg) in the minimum volume of methanol was added. After 17 hours at 5°C, triethylamine (0.1 ml) was added and after 10 mins the solvent was evaporated off. Chromatography on silica gel afforded the amine (32) as a solid (66 mg) $_{max}$ ($CHCl_3$) 3375, 1730 $cm^{-1}$; ppm ($CDCl_3$) 0.23 (6H, s), 0.95 (9H, s), 1.74 (2H, s, exchange) 2.97 (1H, dd, *J* 6.3 and 3Hz), 3.51 (1H, dd, *J* 6.3 and 6Hz), 4.24 (1H, dd, *J* 6 and 3Hz). (Found : $M^+$-H, 199.1281, $C_9H_{19}N_2OSi$ requires *M*-$H^7$ 199.1301).

Example 27

(3S)-1-t-Butyldimethylsilyl-3-N-p-nitrobenzylideneamino azetidin-2-one (33)

(32)

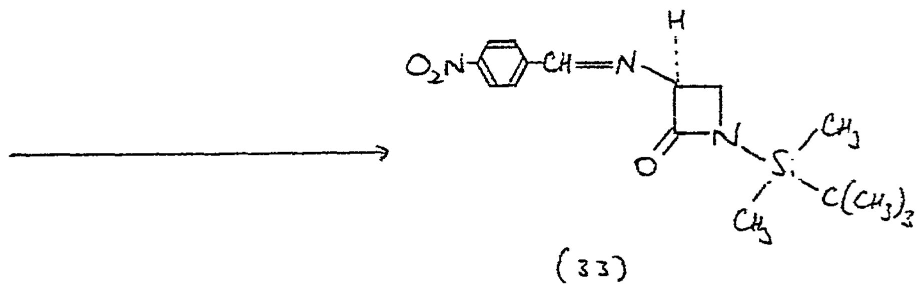

(33)

The amine (32) (2.096 g) was dissolved in toluene (50 ml) and p-nitrobenzaldehyde (1.58 g) added. The solution was vigorously stirred for 18 hours in the presence of 3A molecular sieves. The mixture was filtered through Kieselguhr and the filtrate evaporated. The residue slowly crystallised and was recrystallised from hexane to provide (33) (3.26 g); $[\alpha]^{19}_D$ -205° (c 0.8 in $CHCl_3$) $\nu_{max}$ ($CHCl_3$) 1740, 1635, 1520, and 1350 $cm^{-1}$; $\delta$($CDCl_3$) 0.99 (9H, s), 3.47 (1H, dd, J 6.5 and 3Hz), 3.68 (1H, dd, J 6.5 and 6Hz), 5.0 (1H, m), 7.92 (2H, s, J 8Hz), 8.28 (2H, s, J 8Hz), and 8.55 (1H, slightly broadened s, J ca 0.5Hz).

## Demonstration of Effectiveness

In a standard microtitre (MIC) test, the compound of Example 18 gave the following data:

| Organism | MIC(μg/ml) |
|---|---|
| E. coli ESS | 5.0 |
| E. coli JT425 | 25 |
| K. aerogenes A | 25 |

CLAIMS



1. A β-lactam antibiotic having a sulphonic acid substituent or salt thereof at the 1-position and a hydroxymethylsubstituent at the 3-position.

2. A compound of the formula (I):

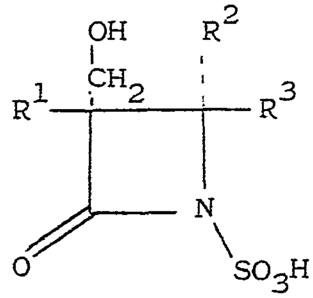

or a salt thereof wherein $R^1$ is an amino, protected amino or carboxylic acylamino group, and $R^2$ and $R^3$ are independently selected from hydrogen or a hydrocarbon group of 1 to 18 carbon atoms.

3. A compound as claimed in claim 2 wherein $R^1$ is a carboxylic acylamino group.

4. A compound as claimed in claim 2 or claim 3 wherein $R^1$ is selected from the subformulae (a) - (e)

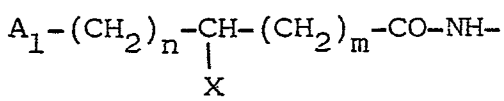 (a)

$A_2$-CO-NH- (b)

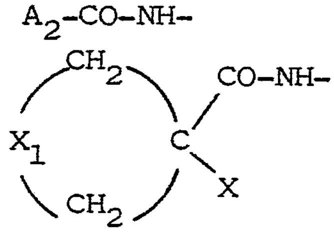 (c)

$A_2$-$X_2$-$(CH_2)_n$-CO-NH- (d)

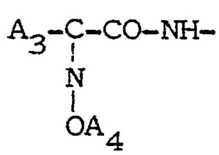 (e)

wherein n is zero, one or two, m is zero, one or two; $A_1$ is $C_{1-6}$ alkyl, $C_{3-6}$ cycloalkyl, $C_{4-7}$ cycloalkenyl, cyclohexadienyl, aryl or heteroaryl; X is a hydrogen, bromine or chlorine atom, or a carboxylic acid, carboxylate ester, sulphonic acid, tetrazolyl, azido, hydroxy, acyloxy, amino, acylamino, heterocyclylamino, ureido, guanidino or acylureido; $A_2$ is an aromatic group; $X_1$ is a $CH_2OCH_2$, $CH_2SCH_2$ or $(CH_2)_n$ group, $X_2$ is an oxygen or sulphur atom; $A_3$ is an aryl or heteroaryl group; and $A_4$ is hydrogen, $C_{1-6}$ alkyl, $C_{3-6}$ cycloalkyl, arylaminocarbonyl, $C_{1-6}$ alkylaminocarbonyl, $C_{1-6}$ alkanoyl, $C_{1-6}$ alkoxycarbonyl, $C_{2-6}$ alkenyl, carboxy $C_{1-6}$ alkyl, $C_{1-6}$ alkylsulphonyl and di-$C_{1-6}$alkylphosphatomethyl.

5. A compound as claimed in any one of claim 2 to 4 wherein $R^1$ is selected from the sub-formulae (f) and (g):

$$R^4\text{-CH-CO-NH} \quad (f)$$
$$\quad |$$
$$\quad R^5$$

$$R^6\text{-CH-CO-NH} \quad (g)$$
$$\quad |$$
$$\quad R^7$$

wherein $R^4$ is a phenyl, thienyl or phenoxy group; $R^5$ is a hydrogen atom or methyl group; $R^6$ is a phenyl, p-hydroxyphenyl, cyclohexadienyl, or a 5- or 6-membered heteroaryl or heterocyclyl group containing one to three heteroatoms selected from sulphur, oxygen or nitrogen, said group being optionally substituted by one, two or three substituents selected from hydroxy, amino, halo and $C_{1-6}$ alkoxy; and $R^7$ is a hydroxy, amino or carboxylic acid group or a phenyl, methylphenyl, indanyl or $C_{1-6}$ alkyl ester thereof, or is amino, ureido, acylamino or acylureido.

6. A compound as claimed in claim 2 wherein $R^1$ is amino or protected amino.

7. A process for the preparation of a compound as claimed in claim 1 which process comprises the sulphonation of a β-lactam having a hydrogen substituent at the 1 position and a hydroxymethyl substituent at the 3 position.

8. A process for the preparation of a compound as claimed in claim 2 which process comprises the reaction of a compound of the formula (X):

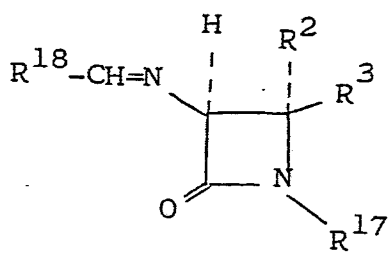

wherein $R^2$ and $R^3$ are as hereinbefore defined, $R^{17}$ is hydrogen, $SO_3H$ or a protecting group, and $R^{18}$ is an optionally substituted aromatic ring; with formaldehyde; and thereafter, if necessary:

i) optionally protecting the $-CH_2OH$ group;
ii) converting $R^{18}$-CH=N to an amino group;
iii) converting said amino group to a carboxylic acylamino group;
iv) converting a compound wherein $R^{17}$ is not $SO_3H$ to a compound wherein $R^{17}$ is $SO_3H$,
v) removing any protecting groups.

9. A pharmaceutical composition comprising a compound as claimed in claim 1 together with a pharmaceutically acceptable carrier.

10. A pharmaceutical composition as claimed in claim 9 which further comprises a β-lactamase inhibitor.

0086563

European Patent Office

# EUROPEAN SEARCH REPORT

Application number

EP 83 30 0234

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 021 678 (TAKEDA YAKUHIN KOGYO K.K.)<br>* Claims 1-3, 6, 7 *<br>--- | 1-7,9, 10 | C 07 D 205/08<br>C 07 D 403/12<br>C 07 D 405/12<br>C 07 D 405/14<br>C 07 D 409/12<br>C 07 D 409/14<br>A 61 K 31/395 |
| D,A | GB-A-2 071 650 (E.R. SQUIBB AND SONS INC.)<br>* Claims 1-10, 14-17, 24-27, 45, 50-57, 81, 105 * & EP - A2 - 0 048 953 (Cat. P,A)<br>--- | 1-7,9 | |
| P,A | EP-A-0 050 965 (TAKEDA CHEMICAL INDUSTRIE, LTD.)<br>* Claims 1, 2 *<br>----- | 1,10 | |
| | | | TECHNICAL FIELDS SEARCHED (Int. Cl. 3) |
| | | | A 61 K 31/395<br>C 07 D 205/00<br>C 07 D 403/02<br>C 07 D 403/12<br>C 07 D 405/00<br>C 07 D 409/00 |

The present search report has been drawn up for all claims

| Place of search | Date of completion of the search | Examiner |
|---|---|---|
| BERLIN | 20-04-1983 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82